# EUROPEAN PATENT SPECIFICATION

(11) **EP 1 642 549 B1**
(45) Date of publication and mention of the grant of the patent: **29.08.2007**
(21) Application number: 05255791.5
(22) Date of filing: 20.09.2005
(51) Int. Cl.: A61F 2/01

(54) **Safety cartridge for retrievable medical filter**
Hülse zum sicheren Einbringen von medizinischen Filtern
Cartouche de sécurité pour un filtre médical

(30) Priority: 30.09.2004 US 955012
(43) Date of publication of application: 05.04.2006
(73) Proprietor: Cordis Corporation, Miami Lakes, FL 33014 (US)
(72) Inventor: Mulder, Rudolf T., 9728 TM Groningen (NL)
(74) Representative: Belcher, Simon James

(56) References cited:
- US-A- 5 188 616
- US-A- 5 846 260
- US-A1- 2004 088 001

## Description

The present invention relates to a safety cartridge for a medical filter which can be placed inside a blood vessel or other body passage for the purpose of intercepting thrombus or particles, which may be optionally retrieved at a later time.

Some types of medical filters are generally known, wherein a single filter element, mesh or member extends across the direction of flow inside a blood vessel. Several features are desirable for medical filters, including non-surgical or percutaneous delivery of the filter to a desired site, and expansion from a preferably small initial size to an expanded working size that matches the anatomy at the desired site. Also, a medical filter should tend to capture a sufficient percentage of thrombus or other particulates, while allowing blood or other fluids to flow freely through the filter.

Another desirable feature is a capability to remain in the desired position for treatment through a period of time, and also to offer the physician the option during that time of leaving the filter in place permanently, or retrieving the filter when no longer needed.

In addition, a medical filter should preferably have a design whereby the filter is stable in the vessel, such that the filter has little or no tendency to "tilt" and may become less effective in capturing thrombus. Prior medical filters may consist of a network of interconnected ribs, which extend substantially in a radial direction in relation to the blood vessel or body passage.
Some medical filters may be used in the vena cava, and in that case may be described as a "vena cava filter."

A medical filter which can be inserted through a percutaneous access point, via a catheter or introducer, and placed in a blood vessel or other body passage. Filters may have features which are not longitudinally symmetrical, such as anchors or barbs which angle toward one end, or a retrieval hook at only one end. Depending on the physician's selection of the access point, the filter may be introduced to a desired site for treatment from opposing directions.

However, it is desirable to introduce the filter with the desired alignment (inserting the desired end in first) regardless of where the selected access point is, and accordingly regardless of which direction of approach to the desired site. As a result, it is desirable to provide a safety cartridge.

Cartridges hold filters in an initial compressed shape during storage and insertion into the patient. A safety cartridge may have labels and/or indicators corresponding to which approach direction is selected, showing which end of the cartridge, and so which end of the filter inside the safety cartridge, to insert first into a catheter or introducer. A safety cartridge may have uniquely shaped first and second ends, which match insertion port(s) on a catheter or introducer which correspond to the selected approach direction. Accordingly, the safety cartridge assists proper orientation of the filter during delivery and treatment.

After insertion from the safety cartridge into the catheter or introducer, a medical filter may be delivered through the catheter or introducer in a radially compressed shape, where it tends to resiliently expand within the blood vessel. The medical filter may tend to trap thrombus or particles, and resist their movement further downstream. The filter may include, in a position of use, an outer shape corresponding to the internal size of the body passage or blood vessel.

Medical filters generally have three types: permanent, temporary or retrievable. A permanent filter is intended for permanent implantation, and a temporary filter is intended for temporary implantation followed by retrieval. In contrast, a retrievable filter offers the physician the option of implanting the filter without initially deciding at that time whether the filter will eventually be retrieved or is to remain permanently. A retrievable filter thus offers the greatest flexibility of medical treatment.

On a temporary or retrievable filter, it is also desirable to provide releasable temporary position stabilizers, to resist a possibility of tilting and to enhance position retention. Some medical filters provide anchors or small barbs, which extend at various angles in radial directions outward from the ribs.

One successful design which is sufficient for a variety of applications is shown in US-6443972, entitled "Vascular Filter" which issued on September 3, 2002. This patent shows many desirable features for filters, including a longitudinally stable central body section, and two filter sections providing multiple filtering action. Other advantageous features are that it is preferably made of a single piece of resilient material, and can be implanted through a catheter without requiring surgery.

US-2004/0088001 discusses a retrievable medical filter. The filter may be designed with one end customized for retrieval and the other end customized for position retention. A cartridge can be provided to ensure proper orientation of the filter during delivery.

US-5188616 relates to a device that can insert its contents, such as a blood filter, in either of two opposite directions into the body of a patient.

According to the present invention, there is provided a medical filter system as defined in appended claim 1.

The filter may preferably have a retrieval structure or hook at one or both ends, of a design that preferably provides multiple hook surfaces for increasing ease of retrieval. A possible filter design is to provide a filter with a hook structure at one end, and cooperating barbs for holding the filter in position, that are arranged to pull out gently if the filter is retrieved in a retrieval direction by pulling on the hook structure with a retrieval snare or other retrieval device.

The term "tubular" is used in its broadest sense, to encompass any structure arranged at a radial distance around a longitudinal axis. Accordingly, tubular includes any structure that (i) is cylindrical or not, such as for example an elliptical or polygonal cross-section, or any other regular or irregular cross-section; (ii) has a different or changing cross-section along its length; (iii) is arranged around a straight, curving, bent or discontinuous longitudinal axis; (iv) has an imperforate surface, or a periodic or other perforate, irregular or gapped surface or cross-section; (v) is spaced uniformly or irregularly, including being spaced varying radial distances from the longitudinal axis; (vi) has any desired combination of length or cross-sectional size.

A medical filter may have a first and second filter section, arranged on either side of a central body section. The body section and the filter sections thus enclose a space. Due to the elongated shape of the medical filter according to the present invention, and the arranging of the first and second section on either side of the body member, the filter has an enhanced filtering effect. In other words, two filtering structures have been created for intercepting thrombus, particulates or other matter moving inside the blood vessel or other body passage.

Filters may also have, when seen in an axial or longitudinal direction, filter sections with the shape of a regular polygon, providing several smaller filtering "cells." The filter sections, as arranged according to an embodiment described above on either side of the tubular body section, may be identical in shape, thereby enhancing the simplicity of the medical filter according to the present invention.

Some medical filters may have been made of a braiding of wire-like elements, or a tubular element also having a series of cuts at places corresponding to the positions of passages. It is thus possible to build various medical filters for use with a safety cartridge according to the present invention, by various techniques and of various materials to obtain the ultimate shape and desired design. Possible junctions of filter components may be formed by melting or welding free ends together, or by employing such a cutting pattern that the basic shape of the medical filter according to the present invention is obtained. It should be noted that the present invention also relates to methods for using a safety cartridge with an implantable filter, as described herein.

The present invention can be used in a method for delivering and deploying a medical filter, comprising the steps of:
providing a catheter introducer system having a proximal and distal end; the catheter having a proximal hub defining a first and second port and defining a lumen extending from the first and second ports to a distal catheter port; the first and second ports being identifiable and having a first and second shape, respectively;
providing a medical filter for delivery through the lumen of the catheter delivery system;
providing a cartridge having a first and second cartridge end, the cartridge having indicators indicating which end is the first end and which end is the second end; the filter being provided and stored in the cartridge in a radially compressed shape, the filter first and second ends each being arranged nearer to the cartridge first and second ends, respectively; wherein the cartridge first and second ends have different shapes corresponding with the first and second ports, respectively;
introducing the catheter distal end to a position near a desired site for treatment, from an access point defining either a first or second direction;
coupling the corresponding end of the cartridge with the selected port, such that the desired end of the filter inside the cartridge points toward the catheter according to which direction is selected; wherein the corresponding shapes of the cartridge ends and the ports provides tactile confirmation that the filter is in the desired orientation; and
pushing the medical filter from the cartridge through the lumen of the catheter and deploying the filter from the catheter distal port.

These and various other objects, advantages and features of the invention will become apparent from the following description and claims, when considered in conjunction with the appended drawings.

Embodiments of the invention will now be described by way of example with reference to the accompanying drawings, in which:
Figures 1 and 2 show side elevational views of opposing sides of a safety cartridge for a medical filter;
Figures 3 and 4 show cross-sectional views, taken along lines 3-3 and 4-4 in Figures 1 and 2;
Figure 5 shows a side elevation view of a medical filter arranged, in an expanded shape;
Figure 6 illustrates an end elevational view along a longitudinal axis of a medical filter, in an expanded shape;
Figures 7 and 8 show partial elevation views of a retrieval hook structure of a medical filter;
Figures 9-12 show partial views of shoulder portions of a medical filter;
Figure 13 is a diagrammatic view of a filter in an initial compressed shape and an expanded deployed shape;
Figure 14 shows a cross-sectional view of a medical filter, implanted in a body passage of a patient;
Figures 15-16 and 23-25 show accessory devices that may be used to deliver implantable filters;
Figures 17-19 show diagrammatic views of a method for delivering and deploying a filter, from one approach direction;
Figures 20-22 show diagrammatic views of a method for delivering and deploying a filter, from an opposing approach direction; and
Figure 26 shows a hub according to the filter system of the present invention for an introducer that may be used to deliver implantable filters.

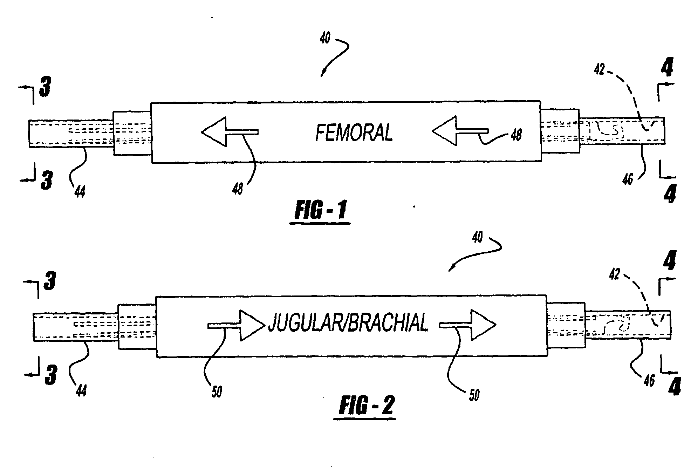

A medical filter 10 may be initially packaged in a compressed state in a safety cartridge 40, shown in Figures 1 and 2. The safety cartridge 40 defines a lumen 42, in which the compressed filter is stored.

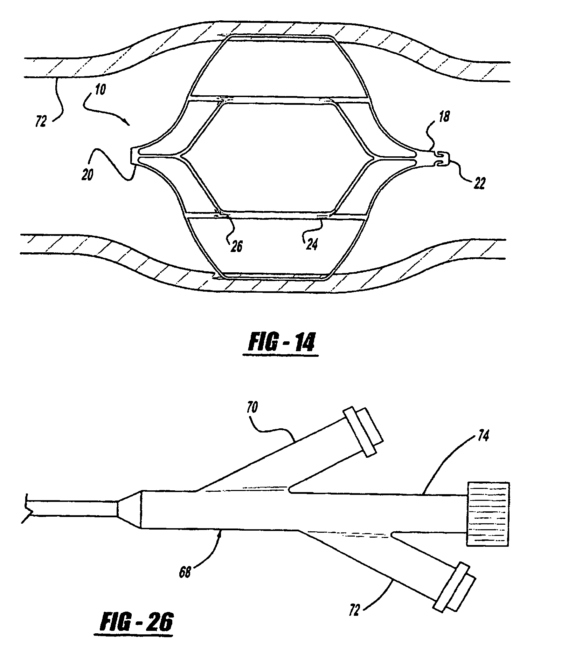

The physician should preferably be able to easily determine which end of the safety cartridge 40, and thus which end of the filter 10, should be inserted first. The filter 10 can accordingly be correctly arranged in the body passage or blood vessel 72, as shown in Figure 14.

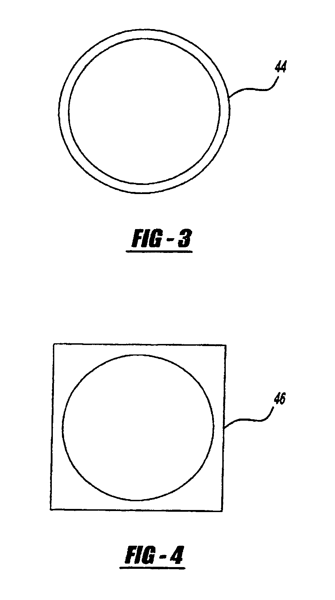

The safety cartridge 40 shown in Figures 1 and 2 also may have a femoral jack 44 and a jugular/brachial jack 46, which preferably have different shapes, to correspond with matching femoral and jugular/brachial access delivery catheter systems, respectively. Such different shapes may for example include, when viewed from each end of the safety cartridge 40, a circle, a square or a triangle. In the safety cartridge shown in the drawings, femoral jack 44 has a circular cross-sectional outer shape, as in Figure 3, and jugular/brachial jack 44 has a square cross-sectional outer shape, as in Figure 4.

The safety cartridge may also have indicators, such as femoral arrows 48 with a label ("FEMORAL") indicating that the femoral jack 44 is to be used for inserting the filter 10 from a femoral access direction, as well as jugular/brachial arrows 50 with a label ("JUGULAR/BRACHIAL") indicating that the jugular jack 46 is to be used for inserting the filter 10 from a jugular access direction.

The combination of these indicators and differently shaped end jacks serves to assist the physician in ensuring that the filter 10 is delivered to the desired site for treatment in the correct orientation.

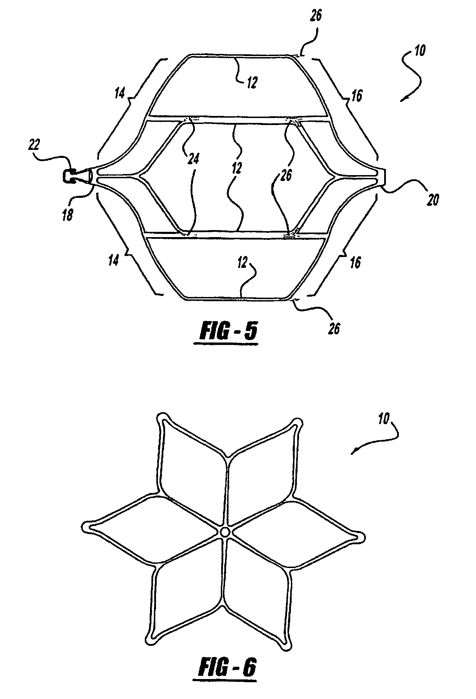

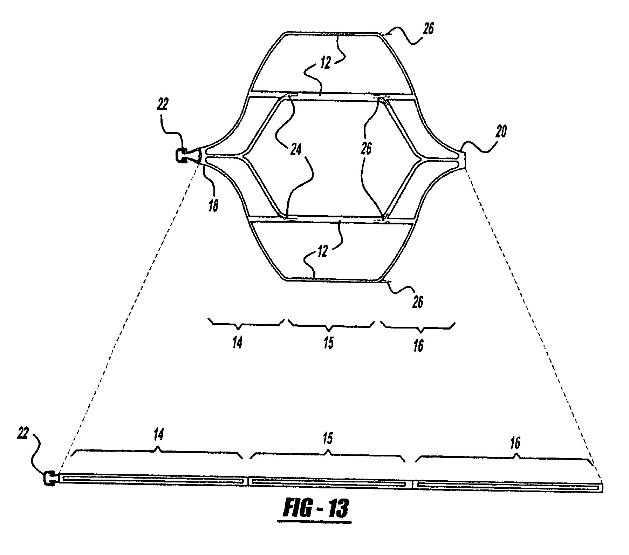

A medical filter is illustrated in Figures 5-14. The filter 10 is preferably made of a resilient material, and tends to expand from an initial compressed shape to an expanded shape, as depicted diagrammatically in Figure 13.

In the expanded shape, the filter 10 may have a series of longitudinal ribs 12, aligned essentially parallel with a longitudinal axis of the filter 10. A plurality of members preferably define a first and second filter section 14 and 16, arranged near a retrieval end of the filter and an insertion end, respectively. A pair of central collars 18 and 20 are also preferably positioned at the retrieval end and the insertion end, respectively. A hook structure 22 is attached to the retrieval collar 18.

In addition, a series of apertures 24 and a corresponding series of anchoring barbs 26 are preferably positioned at shoulders or transitions between a central body section 15 defined by the longitudinal ribs 12 and the filter sections 14 and 16, respectively. In other words, the apertures 24 are located between the ribs 12 and the filter section 14 at the retrieval end, and the barbs 26 are located between the ribs 12 and the filter section 16 at the insertion end of the filter 10.

The filter 10 may be delivered to a desired site for treatment by a delivery catheter 28, which defines a lumen 30 extending between a proximal hub 31 having a first port 32 and a second port 33, each with a hemostatic valve, and the catheter 28 having a distal lumen opening 34. The proximal hub 32 also preferably has a flush lumen tube 36 and a stopcock 38, which may be used for various therapeutic purposes, including flushing the lumen 30 of the delivery catheter 28, injecting radiopaque contrast fluid for viewing on an x-ray video screen, or delivering medications.

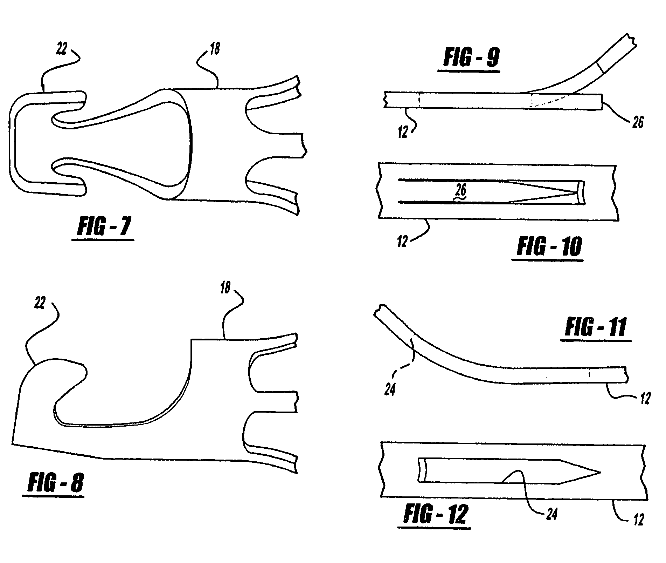

The hook structure 22 of filter 10 may have a T-shape, as shown in Figures 7 and 8, with twin hooks. It is possible that this twin hook structure 22 may improve the ease of retrieving the filter 10.

The barbs or anchors 26 extend in a longitudinal direction, and are exposed when the filter 10 is in an expanded shape. They may be formed as shown in Figures 9 and 10, in which a series of cuts in the ribs 12 both shape the anchor 26 and define an aperture for the anchor 26 when the filter is in a compressed shape.

The apertures 24 may tend to balance stresses in the filter. In other words, anchors 26 may be formed by cutting them out of the ribs, which will tend to bend and define the center section 15 and the filter section 16. Similarly, the presence of apertures 24 may tend to balance stresses in the ribs, causing them to bend in a complementary manner and define the center section 15 and the filter section 14.

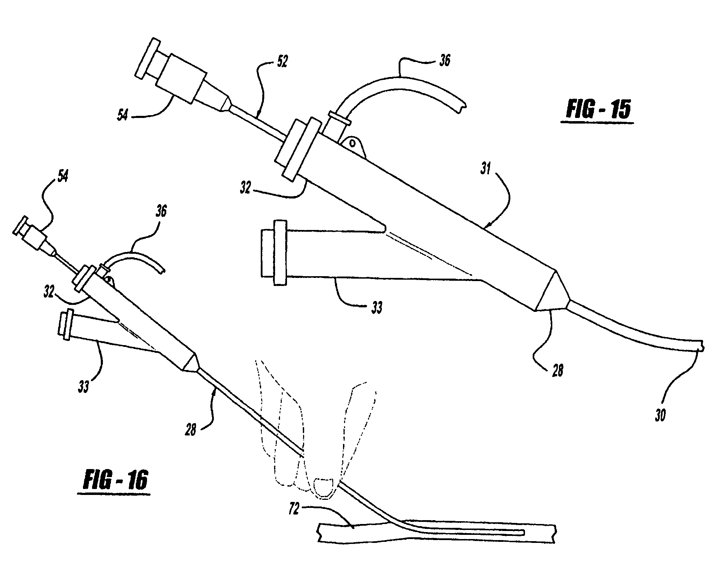

In use, delivery catheter 28 is inserted along a body passage in a patient until distal end 34 is near a desired site for treatment. After filter 10 is disposed within lumen 30 of catheter 28, a push wire 52 may be used to eject the filter 10 from the distal tip 34 of the catheter 28. Push wire 52 shown in Figures 15 and 16 has a proximal hub 54 and a distal end 56. Filter 10 is introduced into the body passage, where the medical filter 10 will resiliently expand after being released from the catheter 28, under the influence of expansive forces inherent to the material of which the medical filter has been made, into the illustrated shape.

Of course, several methods are possible for placing the filter inside lumen 30 of the catheter 28. The filter may simply be placed inside the lumen 30 distal end 34, providing a relatively short distance the filter 10 must be pushed before exiting the catheter 28.

Another possible method for inserting filter 10 at the desired site is to insert it into the lumen 30 proximal end, and then push the filter 10 with a push wire 52 along the entire length of the catheter 28, after the catheter, after the catheter distal end has been advanced to the desired position for treatment.

Another possible aspect is to provide positive feedback on which the end of the filter 10 is inserted first into lumen 30. In other words, it may be desirable to insert one end of the filter into the catheter first, for example when approaching from a femoral access point, and the other end of the first being inserted first at a different access point, for example when approaching a jugular access point. The safety cartridge 40 shown in Figures 1 and 2 may facilitate arranging the filter 10, including a first series of arrows 48 having a first label or indicator such as "FEMORAL", and a second series of arrows 50 having a second label or indicator such as "JUGULAR/BRACHIAL".

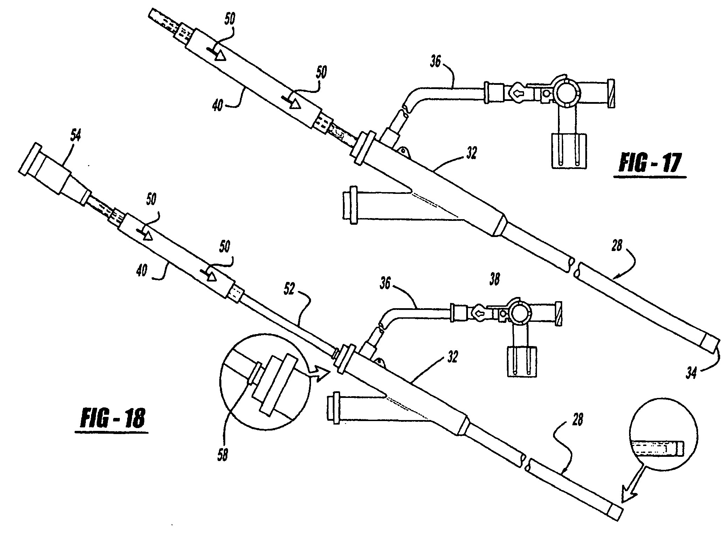

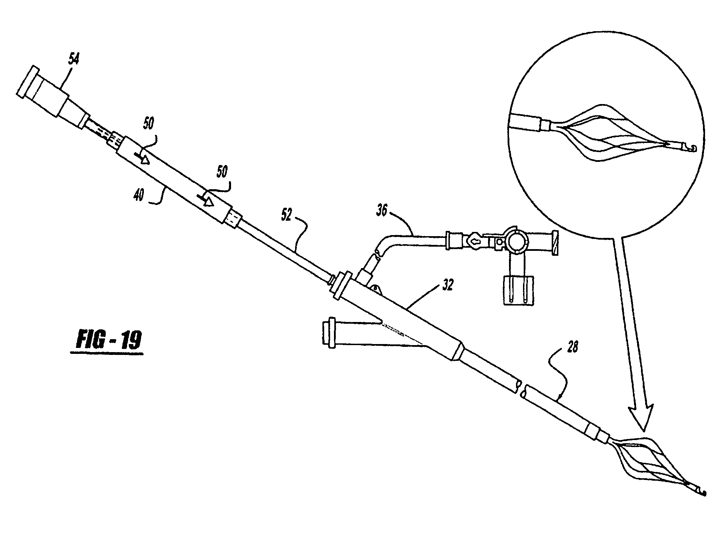

For example, with reference to Figures 17-19, if a jugular approach is selected by the physician, or any other access point where it is desirable that the filter 10 be inserted with the hook structure 22 leading in first, the physician may refer to the "JUGULAR" arrows 50 and insert the corresponding jugular jack 46 at one end of the safety cartridge 40 into the proximal hub and hemostatic valve of a jugular port 33 of the catheter 28. Next, push wire 52 is advanced into the other end of the safety cartridge 40, and through the lumen of the safety cartridge 40 and the catheter 28 until a marker 58 reaches the hemosatic valve. This marker 58 may be provided at a position such that the push wire distal end will have positioned the filter 10 to a point where it has reached the distal end of the catheter 28. At this arrangement, the physician may re-position the assembly to refine the location of the filter 10 just before deploying the filter.

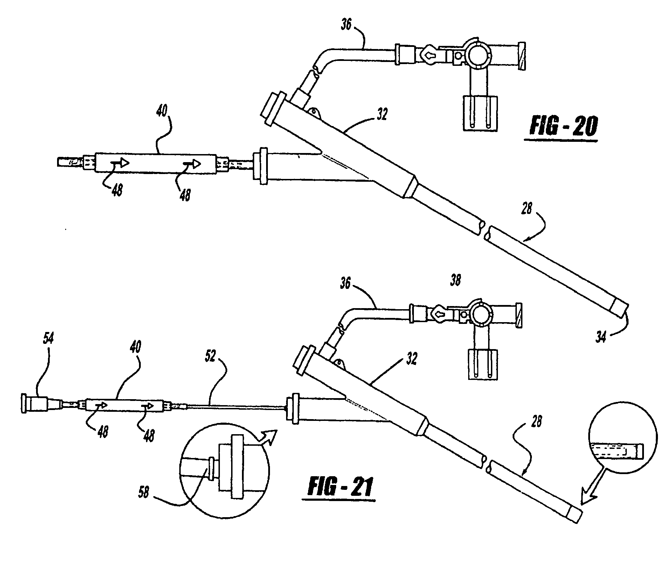

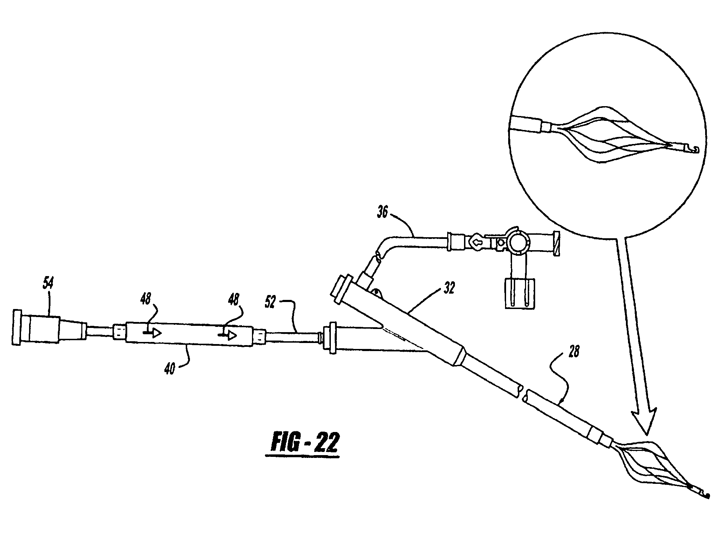

As another example, the physician may choose a femoral access point, or any other access point where the physician desires to insert the filter 10 with the collar 20 end first. In this kind of situation, the physician may choose a catheter 28 having a length suitable for such desired site for treatment, as shown in Figures 20-22.

Accordingly, the physician would in such a case refer to the "FEMORAL" arrows 48, and insert the corresponding femoral jack 44 on the safety cartridge 40 into the proximal hub 32. The push wire 52 is used to advance and deploy the filter 10 as described above.

A hub 68 for a delivery catheter or introducer according to the system of the present invention is shown in Figure 26, which has a first and second filter cartridge insertion port, each of which may have different shapes as described above. In addition, hub 68 has a third central port 74 for insertion of other optional medical devices, such as for example a guidewire. All three ports 70, 72 and 74 may have hemostatic or other types of valves, for resisting leakage of fluids out through the ports.

An additional alternative is to provide dedicated introducer catheters, having hubs with a single port having a selected shape for matching with only one end jack of a safety cartridge. In other words, one introducer with a single-port hub adapted to match one end of the safety cartridge may have a length selected for an approach to a desired site from one direction, for example a "femoral" introducer. Another introducer with a single-port hub adapted to match the other end of the safety cartridge may have a length selected for an approach to a desired site from the other direction, for example a "jugular/brachial" introducer.

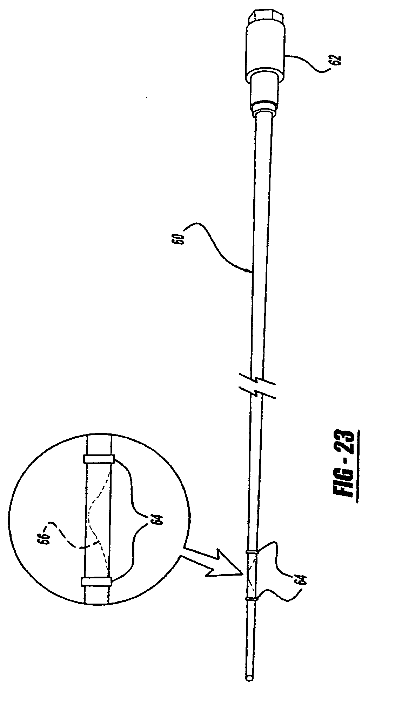

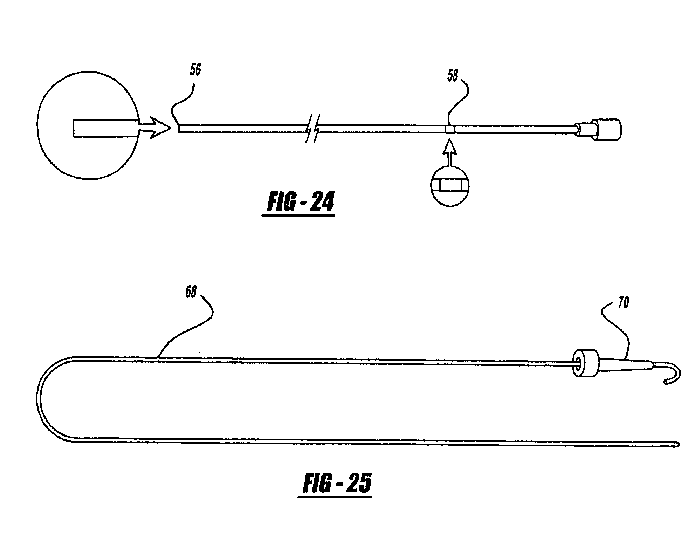

Optional devices that may be used, in particular for example before the catheter 28 is inserted and advanced to the desired site for treatment, are a guidewire 68 and a complementary introducer 70 as shown in Figure 25. The dilator 60 shown in Figure 23 has a proximal hub 62 and spiral sideholes 66, flanked by markers 64. Another optional device that may be used, in particular when the catheter 28 is inserted and advanced to the desired site for treatment, is a dilator 60. The dilator 60 shown in Figure 23 has a proximal hub 62 and spiral sideholes 66, flanked by markers 64.

Medical filters according to the present invention may be made of any suitable material using a variety of methods. One material having the desired characteristics of strength, resilience, flexibility, biocompatibility and endurance is a nickel titanium based shape memory alloy, such as nitinol. Other materials having the desired characteristics may be used, such as stainless steel. Likewise, the manufacturing methods may include providing a tube, and then cutting a pattern into the tube to enable expansion into the desired shape. Various other methods are of course possible, including forming the filter of discrete members and then joining or connecting the members.

In addition to resilient materials such as stainless steel and nitinol, many other materials may also be used for manufacturing a medical filter according to the present invention. By way of alternative, various metals may for instance be used, in which case it is essential that the medical filter assumes the intended shape hereof after having been ejected from the catheter for the purpose of introduction hereof. The medical filter, during introduction, is of course kept in a compressed state, by the catheter. To this end, a configuration may be used decompressing the filter metal due to the elastic properties hereof.

In the axial view, the filter sections on either side of the ribs of the medical filters according to the present invention described above display diamond or polygon shapes. It is also possible to suffice with medical filters of which the filter sections display in axial view a star shape, or any other suitable shape, as long as they intercept blood clots or thrombus successfully. An advantage of this feature is that, after passing the first filter section and the tubular section or the elongated body member, a second chance at interception in the form of an additional filter section has been provided. Also, other shapes of the filter sections in axial view are possible, which shapes will occur to those skilled in the field after reading the present description. The shapes of the filter sections in axial view need not be symmetrical, and may have in principle any suitable appearance.

## Claims

1. A medical filter system for treating a patient, comprising:
a filter (10), a safety cartridge (40) for storing and releasing the filter (10), and an introducer catheter (28);
the safety cartridge (40) having a first and second cartridge end, the safety cartridge (40) having indicators (48, 50) indicating which cartridge end is the first cartridge end and which is the second cartridge end; the first and second cartridge ends having different shapes;
the filter (10) having a first and second filter end, and the filter tends to resiliently expand from a radially compressed shape to an expanded deployed shape;
the filter (10) being provided and stored in the safety cartridge (40) in a radially compressed shape, the first and second filter ends each being arranged nearer to the first and second cartridge ends, respectively; **characterised by**:
the introducer catheter (28) having proximal and distal ends, and a hub (68) at the proximal end defining a first and second introducer port (70, 72);
the first and second introducer ports (70, 72) each having different shapes, each of which match and will accept only one of the first and second cartridge end, respectively;
wherein the first and second cartridge ends and corresponding first and second introducer ports (70, 72) cooperate to assist a physician to introduce the filter (10) in the desired orientation.

2. The medical filter system as set forth in Claim 1, further comprising an additional introducer port (74), for introducing additional medical devices.

## Patentansprüche

1. Medizinisches Filtersystem zur Behandlung eines Patienten, das folgendes umfaßt:
einen Filter (10), eine Sicherungskartusche (40) zum Aufbewahren und Abgeben des Filters (10) und einen Einführkatheter (28);
wobei die Sicherungskartusche (40) ein erstes und ein zweites Kartuschenende auf weist, die Sicherungskartusche (40) zwei Indikatoren (48, 50) aufweist, die anzeigen, welches Kartuschenende das erste Kartuschenende ist und welches das zweite Kartuschenende ist; wobei das erste und das zweite ISariuschenende unterschiedliche Formen aufweisen;
wobei der Filter (10) ein erstes und ein zweites Filterende aufweist und der Filter dazu neigt, sich elastisch aus einer radialen zusammengezogenen Form in eine ausgedehnte entfaltete Form auszudehnen;
der Filter (10) in der Sicherungskartusche (40) in einer radial zusammengefalteten Form bereitgestellt und aufbewahrt wird, wobei das erste und das zweite Filterende jeweils näher zu dem ersten bzw. zweiten Kartuschenende angeordnet sind; **dadurch gekennzeichnet, daß**
der Einführkatheter (28) ein proximales und ein distales Ende aufweist und ein Hub (68) an dem proximalen Ende eine erste und eine zweite Einführöffnung (70, 72) definiert;
die erste und zweite Einführöffnung (70, 72) jeweils unterschiedliche Formen aufweisen, von denen jede nur zu dem ersten oder dem zweiten Kartuschenende paßt und dieses aufnimmt;
wobei das erste und zweite Kartuschenende und die entsprechende erste und zweite Einführöffnung (70, 72) zusammenwirken, um den Mediziner beim Einführen des Filters (10) in der gewünschte Orientierung zu unterstützen.

2. Medizinisches Filtersystem nach Anspruch 1, das weiterhin eine zusätzliche Einführöffnung (74) aufweist zum Einführen zusätzlicher medizinischer Vorrichtungen.

## Revendications

1. Système de filtre médical pour traiter un patient, comprenant :
un filtre (10), une cartouche de sécurité (40) pour stocker et libérer le filtre (10), et un cathéter introducteur (28);
la cartouche de sécurité (40) comportant une première et une seconde extrémités de cartouche, la cartouche de sécurité (40) comportant des indicateurs (48, 50) indiquant quelle extrémité de cartouche est la première extrémité de cartouche et quelle est la seconde extrémité de cartouche ; la première et la seconde extrémités de cartouche ayant des formes différentes ;
le filtre (10) comportant une première et une seconde extrémités de filtre, et le filtre a tendance à s'étendre de manière élastique depuis une forme radialement comprimée jusqu'à une forme étendue déployée ;
le filtre (10) étant présenté et stocké dans la cartouche de sécurité (40) dans une forme radialement comprimée, la première et la seconde extrémités de filtre étant disposées le plus près possible de la première et de la seconde extrémités de cartouche, respectivement, **caractérisé par**:
le cathéter introducteur (28) présentant des extrémités proximale et distale, et un raccord (68) au niveau de l'extrémité proximale définissant un premier et un second orifices introducteurs (70, 72) ;
les premier et second orifices introducteurs (70, 72) ayant chacun une forme différente, chacun d'entre eux correspond à et va admettre seulement l'une de la première ou de la seconde extrémité de cartouche, respectivement ;
dans lequel la première et la seconde extrémités de cartouche correspondant aux premier et second orifices introducteurs (70, 72) concourent à permettre à un médecin d'introduire le filtre (10) dans la direction souhaitée.

2. Système de filtre médical selon la revendication 1, comprenant en outre un orifice introducteur supplémentaire (74), pour introduire des dispositifs médicaux supplémentaires.
